# EUROPÄISCHE PATENTANMELDUNG

(11) **EP 3 563 880 A1**
(43) Veröffentlichungstag der Anmeldung: **06.11.2019**
(21) Anmeldenummer: 18170669.8
(22) Anmeldetag: 03.05.2018
(51) Int. Cl.: A61L 27/04, A61L 27/08, A61L 27/42, A61L 27/58, A61L 31/02, A61L 31/12, A61L 31/14

(54) **RESORBIERBARES IMPLANTATMATERIAL AUS MAGNESIUM ODER EINER MAGNESIUMLEGIERUNG**

(71) Anmelder: Helmholtz-Zentrum Geesthacht Zentrum für Material- und Küstenforschung GmbH, 21502 Geesthacht (DE)
(72) Erfinder: Dieringa, Hajo, 21335 Lüneburg (DE)
(74) Vertreter: Uexküll & Stolberg

(57) **Zusammenfassung**

Die vorliegende Erfindung betrifft ein resorbierbares Implantatmaterial aus Magnesium oder einer Magnesiumlegierung und ein Verfahren zur Herstellung desselben. Nachteil bekannter resorbierbarer Implantate ist, dass deren Resorption bisher nur mittels Röntgen- oder CT-Untersuchungen verfolgt werden kann. Erfindungsgemäß wird ein resorbierbares Implantatmaterial bereitgestellt, das homogen verteilt fluoreszierende Nanodiamanten in einer Matrix aus Magnesium oder einer Magnesiumlegierung umfasst. Floureszierende Nanodiamanten sind für den Organismus unbedenklich und bieten aufgrund von Stickstofffehlstellen (Nitrogen-Vacancy-Zentren, NV-Zentren) eine stabile Emission im nahen Infrarotbereich. Dies ermöglicht die Detektion des Implantatmaterials im Blutplasma des Patienten.

Das erfindungsgemäße resorbierbare Implantatmaterial wird erfindungsgemäß durch ein Verfahren hergestellt, bei dem Magnesium oder eine Magnesiumlegierung aufgeschmolzen wird, zu der Schmelze Nanodiamanten gegeben werden und die mit Nanodiamanten versehene Schmelze aus Magnesium oder einer Magnesiumslegierung einer Ultraschallbehandlung unterzogen wird.

## Beschreibung

### GEBIET DER ERFINDUNG

Die vorliegende Erfindung betrifft ein Implantatmaterial aus Magnesium oder einer Magnesiumlegierung und ein Verfahren zur Herstellung desselben.

### HINTERGRUND DER ERFINDUNG

Sowohl in der Veterinär- als auch in der Humanmedizin werden derzeit zur Versorgung von Frakturen gewichtstragender langer Röhrenknochen Metallimplantate aus medizinischem Stahl oder Titan verwendet. Diese Implantate sind in ihrem mechanischen Verhalten jedoch rigider als Knochen, was zum Phänomen der Belastungsabschirmung ("stress-shielding") führen kann. Aus diesen und anderen Gründen werden entsprechende Implantate in der Regel nach Funktionserfüllung wieder entfernt, was für den Patienten durch die notwendige Narkose und das erneute Gewebetrauma belastend sein kann.

Resorbierbare Implantate werden zunehmend zur Frakturversorgung interessant. Ziel ist es, dass die Implantate mit zunehmender Festigkeit des heilenden Knochens eine Belastungsanpassung durch langsame Abnahme ihre Stabilität erfahren. Der Einsatz der bisher zur Verfügung stehenden resorbierbaren Implantate aus unterschiedlichen Polymeren gelingt aufgrund ihrer niedrigen Festigkeiten am belasteten Knochen nicht optimal. Dagegen weisen Magnesium und seine Legierungen im Vergleich zu anderen metallischen Implantatmaterialien ein den Knochen ähnliches Elastizitätsmodul und günstige Zug-und Druckfestigkeit auf. Magnesium und seine Legierungen besitzen höhere Festigkeiten und einen größeren Elastizitätsmodul als resorbierbare Polymere und stehen daher im Fokus der wissenschaftlichen Forschung. Bioresorbierbare Implantate, insbesondere aus Magnesium oder einer Magnesiumlegierung zur Behandlung von Knochenfrakturen sind beispielsweise aus der EP 2 318 057 B1 und darin zitierten Druckschriften oder der DE 10 2005 060 203 A1 bekannt.

Resorbierbare Implantate werden nicht nur zur Frakturversorgung eingesetzt. Heutzutage werden Implantate aus Magnesium und seinen Legierungen besonders häufig als Stents eingesetzt, die zur Behandlung von Stenosen (Gefäßverengungen) dienen. Stents weisen ein rohrförmiges oder hohlzylinderförmiges Grundgitter auf, dass an beiden Längsenden offen ist. Das rohrförmige Grundgitter einer derartigen Endoprothese wird in das zu behandelnde Gefäß eingesetzt und dient dazu, das Gefäß zu stützen. Biodegradable Stents aus Magnesium oder Magnesiumlegierung sind beispielsweise aus der EP 2 198 898 B1 und darin zitierten Druckschriften bekannt.

Nachteil der bekannten Implantate ist jedoch, dass deren Resorption bisher nur mittels Röntgen- oder CT-Untersuchungen verfolgt werden kann. Diese Untersuchungen sind vergleichsweise aufwendig und kostenintensiv. Aufgabe der vorliegenden Erfindung ist es, ein Implantatmaterial aus Magnesium oder einer Magnesiumlegierung und ein Verfahren zur Herstellung desselben zur Verfügung zu stellen, dessen Resorption im Körper des Patienten auf einfache Weise verfolgt werden kann, ohne dass Röntgen- oder CT-Untersuchungen erforderlich sind.

### ZUSAMMENFASSUNG DER ERFINDUNG

Gelöst wird die Aufgabe durch ein Implantatmaterial gemäß Anspruch 1, das homogen verteilt fluoreszierende Nanodiamanten in einer Matrix aus Magnesium oder einer Magnesiumlegierung umfasst. Gelöst wird die Aufgabe auch durch ein Verfahren zur Herstellung eines Implantatmaterials gemäß Anspruch 8, bei dem Magnesium oder eine Magnesiumlegierung aufgeschmolzen wird, zu der Schmelze Nanodiamanten gegeben werden und die mit Nanodiamanten versehene Schmelze aus Magnesium oder einer Magnesiumslegierung einer Ultraschallbehandlung unterzogen wird.

### DETAILLIERTE BESCHREIBUNG DER ERFIDUNG

Floureszierende Nanodiamanten (FNDs) sind beispielsweise aus K. Merchand und S.K. Sarkar, IEEE JOURNAL OF SELECTED TOPICS IN QUANTUM ELECTRONICS, VOL. 22, NO. 3, MAY/JUNE 2016 als Proteinmarker bekannt. Diese werden bislang in der Forschung zum Sichtbarmachen von biologischen Zellprozessen eingesetzt. Floureszierende Nanodiamanten sind für den Organismus unbedenklich und bieten aufgrund von Stickstofffehlstellen (Nitrogen-Vacancy-Zentren, NV-Zentren) eine stabile Emission im nahen Infrarotbereich.

Das NV-Zentrum im Diamant wird von einem Stickstoffatom, das ein Kohlenstoffatom im Diamantgitter substituiert, und einer direkt benachbarten Leerstelle im Diamantgitter gebildet. Es kann in zwei unterschiedlich geladenen Zuständen vorliegen, dem elektrisch neutralen NV° und dem einfach negativ geladenen NV⁻. Das NV-Zentrum im Diamant besitzt herausragende photophysikalische Eigenschaften. Die Fluoreszenzübergänge vom angeregten Zustand in den Grundzustand liegen beim NV⁻-Zentrum bei 1,945 eV entsprechend einer Fluoreszenzwellenlänge von 638 nm, beim NV⁰-zentrum bei 2,156 eV entsprechend einer Fluoreszenzwellenlänge von 575 nm. Durch die Kopplung an Phononen im Kristall kann es zur Verbreiterung des Fluoreszenzspektrums kommen. Die Herstellung von fluoreszierenden Nanodiamanten ist beispielsweise in der US 2014/0065424 oder der WO 2017/108655 A1 beschrieben, auf die hier vollständig Bezug genommen wird.

Fluoreszierende Nanodiamanten sind beispielsweise auch von der Firma Sigma-Aldrich Chemie GmbH, Steinheim, Deutschland, kommerziell erhältlich.

Das erfindungsgemäße Implantatmaterial aus Magnesium oder einer Magnesiumlegierung, das homogen verteilt fluoreszierende Nanodiamanten enthält, kann durch Gießen hergestellt werden. Danach kann es stranggepresst werden oder mittels pulvermetallurgischer Verfahren wie der MIM-Technologie zu Implantatkörpern verarbeitet werden. Mit der Resorption des Implantatmaterials im Körper des Patienten gelangen die fluoreszierenden Nanodiamanten in den Blutkreislauf, wo sie mittels Fluoreszenzspektroskopie oder auf andere Weise nachgewiesen werden können. Die fluoreszierenden Nanodiamanten werden nach und nach aus dem Körper ausgeschieden. Auf diese Weise erhält man ein An-/Abflutungsprofil im Plasma, das nach Kalibrierung Rückschlüsse auf die Resorption des Implantatmaterials zulässt.

Sofern eine Magnesiumlegierung als Matrixmaterial verwendet wird, werden bevorzugt Legierungselemente verwendet, die als nicht gesundheitsgefährdend gelten. Bevorzugt werden Magnesiumlegierungen mit Legierungselementen verwendet, die ausgewählt sind aus der Gruppe bestehend aus Lithium, Calcium, Kalium, Strontium, Barium, Scandium, Yttrium, Lanthan, Praseodym, Neodym, Samarium, Europium, Gadolinium, Dysprosium, Silizium, Kupfer, Zink, Gallium, Gold, Silber, Bismut, Eisen und Kombinationen davon. Bevorzugter werden Magnesiumlegierungen verwendet, wie sie in DE 10 2016 007 176 A1, oder DE 10 2016 119 227 A1 beschrieben sind, auf die hier vollständig Bezug genommen wird.

Erfindungsgemäß wird das Implantatmaterial hergestellt, indem Magnesium oder eine Magnesiumlegierung aufgeschmolzen wird, zu der Schmelze Nanodiamanten gegeben werden und die mit Nanodiamanten versehene Schmelze aus Magnesium oder einer Magnesiumlegierung einer Ultraschallbehandlung unterzogen wird.

Ein derartiges Verfahren zur homogenen Verteilung von Nanopartikeln in einer Schmelze aus Magnesium oder einer Magnesiumlegierung wird in dem Artikel von H. Dieringa et al. "Ultrasound Assisted Casting of an AM60 Based Metal Matrix Nanocomposite, Its Properties, and Recyclability" in Metals 2017, 7, 338 beschrieben, auf den hier vollständig Bezug genommen wird.

Bei einem bevorzugten Verfahren zur Herstellung des erfindungsgemäßen Implantatmaterials wird Magnesium oder eine Magnesiumlegierung bevorzugt in einem ersten Schritt in einer in einem Ofen befindlichen Kokille unter Schutzgas und unter Rühren aufgeschmolzen, in einem zweiten Schritt wird die Schmelze mit den Nanodiamanten versetzt und in einem dritten Schritt werden die in die Schmelze eingebrachten Nanodiamanten mittels einer Sonotrode dispergiert und deagglomeriert. Es ist ferner bevorzugt, dass die Kokille mit der Schmelze nach Herausnehmen des Rührers und der Sonotrode in ein Wasserbad getaucht wird. Somit erfolgt eine Erstarrung der Schmelze von "unten nach oben", wodurch eine Lunkerbildung vermieden wird.

Das so hergestellte Implantatmaterial kann im Anschluss auf gewöhnliche Weise weiterverarbeitet werden. Beispielsweise kann das Implantatmaterial wieder aufgeschmolzen und anschließend in die gewünschte Form zur Bildung eines Implantatkörpers gegossen werden. Das Material kann auch stranggepresst werden, um aus dem Extrudat Implantate zu fertigen. Alternativ kann das Implantatmaterial zu Pulver weiterverarbeitet und mittels Metallspritzgiessen ("metal injection moulding", MIM) zu einem Implantatkörper weiterverarbeitet werden.

Das erfindungsgemäße Implantatmaterial umfasst bevorzugt homogen verteilte fluoreszierende Nanodiamanten in einer Matrix aus Magnesium oder einer Magnesiumlegierung in einer Menge von 0,1 bis 5 Gew.-%, bevorzugt 0,5 bis 1,5 Gew.-% auf Basis des Gewichts an Magnesium bzw. Magnesiumlegierung. Die Nanodiamanten haben bevorzugt eine Teilchengröße von 1 bis 20 nm, bevorzugt 3 bis 8 nm.

Wie beschrieben wird zur Herstellung des erfindungsgemäßen Implantatmaterials das Magnesium oder die Magnesiumlegierung bevorzugt in einem ersten Schritt in einer in einem Ofen befindlichen Kokille unter Schutzgas und unter Rühren aufgeschmolzen, wie in H. Dieringa et al. "Ultrasound Assisted Casting of an AM60 Based Metal Matrix Nanocomposite, Its Properties, and Recyclability" in Metals 2017, 7, 338 beschrieben. Die Schmelze wird vorzugsweise mechanisch gerührt, vorzugsweise bei 150 bis 250 UpM. Anschließend werden die fluoreszierenden Nanodiamanten der Schmelze zugefügt. Nach Zugabe der fluoreszierenden Nanodiamanten wird die Schmelze mit Ultraschall behandelt. Dazu wird bevorzugt eine Sonotrode in die Schmelze eingebracht. Die Ultraschallbehandlung findet bevorzugt über eine Zeitspanne von 1 Min. bis 10 Min., bevorzugter 2 Min. bis 5 Min. statt.

Es ist bevorzugt, dass nach dem Mischen und der Ultraschallbehandlung der Rührer und die Sonotrode entfernt werden und die Kokille langsam aus dem Ofen in ein Wasserbad abgesenkt wird, wo die Schmelze erstarrt.

Das so hergestellte Implantatmaterial kann im Anschluss auf gewöhnliche Weise weiterverarbeitet werden. Beispielsweise kann das Implantatmaterial wieder aufgeschmolzen und anschließend in die gewünschte Form gegossen werden, um einen metallischen Implantatkörper zu liefern. Das erfindungsgemäße Implantatmaterial kann auch stranggepresst werden und aus dem Extrudat kann ein Implantatkörper gefertigt werden.

Das erfindungsgemäße Implantatmaterial kann auch mit Hilfe der MIM-Technologie zu einem metallischen Implantatkörper verarbeitet werden. Mit Hilfe der MIM-Technologie lassen sich kleine, komplexe und präzise geformte Metallbauteile endformnah fertigen. Die MIM-Technologie gehört zu den sogenannten pulvermetallurgischen Verfahren, bei denen kein massiver Metallkörper, sondern feines Metallpulver als Ausgangsmaterial für das herzustellende Bauteil verwendet wird. MIM steht dabei für "Metal Injection Moulding" (Metallpulverspritzguss). Beim MIM-Verfahren wird das Metallpulver durch Zusatz von thermoplastischen Bindemitteln fließfähig gemacht und das fließfähige Gemisch in eine Spritzgussform eingebracht. Nach der Formgebung wird der Bindemittelanteil wieder entfernt und das Bauteil gesintert. Magnesiumbauteile können mithilfe der MIM-Technologie nach dem in M. Wolff et. al. "Magnesium powder injection moulding for biomedical application", Powder Metallurgy, 2014 (Vol. 57, No. 5), 331-340 beschriebenen Verfahren hergestellt werden, auf das hier vollständig Bezug genommen wird.

Das Bindemittel sorgt bei Anwendung der MIM-Technologie für eine temporäre Verbindung während des Urformens bzw. der Formgebung und sichert die Stabilität des Bauteils bis zur endgültigen Kompaktierung des Metallpulvers durch Sintern. Ein Teil des Bindemittels wird in der Regel bereits vor der Sinterung, zum Beispiel Mithilfe eines Lösungsmittels entfernt (Lösemittelentbinderung). Der Rest des Bindemittels zersetzt sich bei der thermischen Entbinderung bei Temperaturen von etwa 300°C bis 500°C und entweicht gasförmig.

## Patentansprüche

1. Implantatmaterial, das homogen verteilt fluoreszierende Nanodiamanten in einer Matrix aus Magnesium oder einer Magnesiumlegierung umfasst.

2. Implantatmaterial nach Anspruch 1, wobei die homogen verteilten fluoreszierenden Nanodiamanten in einer Menge von 0,1 bis 5 Gew.-% auf Basis des Gewichts des Magnesiums bzw. der Magnesiumlegierung in der Matrix vorliegen.

3. Implantatmaterial nach Anspruch 2, wobei die homogen verteilten fluoreszierenden Nanodiamanten in einer Menge von 0,5 bis 1,5 Gew.-% auf Basis des Gewichts des Magnesiums bzw. der Magnesiumlegierung in der Matrix vorliegen.

4. Implantatmaterial nach einem der vorgehenden Ansprüche, wobei die fluoreszierenden Nanodiamanten eine Teilchengröße von Teilchengröße von 1 bis 20 nm aufweisen.

5. Implantatmaterial nach Anspruch 4, wobei die fluoreszierenden Nanodiamanten eine Teilchengröße von Teilchengröße von 3 bis 8 nm aufweisen.

6. Verfahren zur Herstellung eines Implantatmaterials gemäß einem der vorgehenden Ansprüche, bei dem Magnesium oder eine Magnesiumlegierung aufgeschmolzen wird, zu der Schmelze Nanodiamanten gegeben werden und die mit Nanodiamanten versehene Schmelze aus Magnesium oder einer Magnesiumslegierung einer Ultraschallbehandlung unterzogen wird.

7. Verfahren nach Anspruch 6, bei dem das Magnesium oder die Magnesiumlegierung in einem ersten Schritt in einer in einem Ofen befindlichen Kokille unter Schutzgas und unter Rühren aufgeschmolzen wird, die Schmelze mechanisch gerührt wird, anschließend die fluoreszierenden Nanodiamanten der Schmelze zugefügt werden und nach Zugabe der fluoreszierenden Nanodiamanten wird die Schmelze mit Ultraschall behandelt wird.

8. Verfahren nach Anspruch 7, wobei die Ultraschallbehandlung mittels einer in die Schmelze eingebrachten Sonotrode erfolgt.

9. Verfahren nach einem der Ansprüche 6 bis 8, bei dem die Ultraschallbehandlung über eine Zeitspanne von 1 Min. bis 10 Min. stattfindet.

10. Verfahren nach Anspruch 9, bei dem die Ultraschallbehandlung über eine Zeitspanne von 2 Min. bis 5 Min. stattfindet.

11. Verfahren nach einem der Ansprüche 7 bis 10, bei dem die Kokille nach der Ultraschallbehandlung in ein Wasserbad überführt wird, wo die Schmelze erstarrt.

12. Verfahren nach einem der Ansprüche 6 bis 11, bei dem das Implantatmaterial wieder aufgeschmolzen und anschließend in die gewünschte Form gegossen werden, um ein metallisches Implantat zu liefern.

13. Verfahren nach einem der Ansprüche 6 bis 11, bei dem das Implantatmaterial extrudiert wird und das Extrudat als Grundstoff für die Fertigung eines Implantates dient.

14. Verfahren nach einem der Ansprüche 6 bis 11, bei dem das Implantatmaterial mittels MIM-Technologie in ein metallisches Implantat überführt wird.
